(19) Europäisches Patentamt
European Patent Office
Office européen des brevets

(11) Veröffentlichungsnummer: **0 048 345**
**B1**

# (12) EUROPÄISCHE PATENTSCHRIFT

(45) Veröffentlichungstag der Patentschrift: **21.09.83**

(51) Int. Cl.³: **C 07 C 103/52,** A 23 L 1/236, C 07 C 102/00

(21) Anmeldenummer: **81106544.0**

(22) Anmeldetag: **22.08.81**

## (54) Verfahren zur Herstellung eines alpha-L-Aspartyl-L-Phenylalanin-Alkyl-Esters.

(30) Priorität: **19.09.80 US 185721**

(43) Veröffentlichungstag der Anmeldung: **31.03.82 Patentblatt 82/13**

(45) Bekanntmachung des Hinweises auf die Patenterteilung: **21.09.83 Patentblatt 83/38**

(84) Benannte Vertragsstaaten: **AT BE CH DE FR GB IT LI NL SE**

(56) Entgegenhaltungen:
**US-A-3 475 403**
**US-A-3 933 781**

**Chemical Abstracts Band 49, Nr. 10, 1955 Columbus, Ohio, USA, LEACH et al. "The preparation of some dipeptides containing asparagine, aspartic acid, and glutamine" Spalte 6832, Abstract Nr. 6832 b-e.**

**HOUBEN-WEYL « Methoden der organischen Chemie» 4. Auflage, Band 15/2, 1974, GEORG THIEME VERLAG, Stuttgart, Seiten 170 bis 183**

**Die Akte enthält technische Angaben, die nach dem Eingang der Anmeldung eingereicht wurden und die nicht in dieser Patentschrift enthalten sind.**

(73) Patentinhaber: **CHIMICASA GMBH, Wiesentalstrasse 81, CH-7000 Chur (CH)**

(72) Erfinder: **Sampathkumar, Prathivadibhayankaram S., 23 Rockaway Boulevard, Parsippany New Jersey 07054 (US)**
Erfinder: **Dwivedi, Basant K., 3 Farm Road, Randolph New Jersey (US)**

(74) Vertreter: **Hach, Hans Karl, Dr., Tarunstrasse 23, D-6950 Mosbach-Waldstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung eines Alpha-L-Aspartyl-L-Phenylalanin-Alkyl-Esters

Die Erfindung betrifft ein Verfahren zur Herstellung eines Alpha-L-Aspartyl-L-Phenylalanin-Alkyl-Esters.

Gemäss der US-PS 3.475.403 erfolgt die Herstellung des Beta-Benzyl-N-Benzyloxycarbonyl-L-Aspartyl-L-Tyrosin-Methylesters anhand Reaktion von N-Benzyloxycarbonyl-L-Asparaginsäure-Alpha-p-Nitrophenol und Beta-Benzylester und L-Tyrosin-Methylester. Laut US-Patent 3.933.781 erfolgt die Herstellung des N-Formyl-Alpha-L-Aspartyl-L-Phenylalanins unter Verwendung von L-Phenylalanin und N-Formyl-L-Asparaginsäureanhydrid mit anschliessender Deformylierung und Veresterung zwecks Erhalt der Methylester-Verbindung.

Die nach dem Stand der Technik vorliegenden Methoden erfordern eine Reihe von Prozessstufen sowie den Einsatz verschiedener, kostspieliger Reagenzien.

Aus Houben-Weyl's «Methoden der organischen Chemie», 4. Auflage, Band 15/2 (1974), Seite 170 bis 183, ist eine allgemeine Vorschrift zur Herstellung von Peptiden nach der Kohlensäure-Anhydrid-Methode bekannt. Die Anwendung dieses bekannten Verfahrens unter Berücksichtigung der in dieser Literaturstelle angegebenen speziellen Synthesebeispiele führt aber zu so geringer Ausbeute, dass darauf eine Produktion nicht gestützt werden kann.

Aufgabe der Erfindung ist es, ein Verfahren der eingangs genannten Art dahingehend zu verbessern, dass möglichst kostengünstig und mit möglichst hoher Ausbeute Alpha-L-Aspartyl-L-Phenylalanin-Alkyl-Ester und insbesondere der entsprechende Methylester herstellbar ist und dass dabei die Racemisierung und die Bildung des nicht süssenden Isomeren vermieden werden kann.

Die Erfindung ist gekennzeichnet durch:

a) Reaktion eines Dialkali-Salzes der L-Asparaginsäure mit geschützter Aminogruppe mit einer organischen Halogen-Ester-Verbindung zur Bildung eines Monoalkali-Salzes einer Gemischten-Anhydrid-Verbindung;

b) Kondensation der Gemischten-Anhydrid-Verbindung mit einem Alkylester von L-Phenylalanin unter Alkalibedingungen und Reduzierung des pH-Wertes nach der Kondensation auf Säurebedingungen, um die Amino- und Carboxylgruppen freizusetzen, zur Bildung eines Gemisches, das im wesentlichen aus dem Alpha- und Beta-Alkylester des L-Aspartyl-L-Phenylalanins besteht; und

c) Abtrennung des Alpha-Isomeren aus dem Alpha- und Beta-Gemisch.

Bei der Gemischten-Anhydrid-Verbindung handelt es sich um das Monoalkali-Salz des Mischanhydrids aus aminogruppengeschützter Asparaginsäure und einer O-Alkylcarbonsäure.

Alpha-Dipeptid-Alkylester und insbesondere der Methylester, welcher die Süssstoff-Verbindung darstellt, können unmittelbar und in hoher Ausbeute durch die Reaktion eines Dialkalimetallsalzes der Asparaginsäure, in welchem die Aminogruppe geschützt ist, mit einem geeigneten Anhydrid-Partner, wie beispielsweise eine Halogenester-Verbindung, zur Bildung eines Monoalkali-Salzes einer Gemischten-Anhydrid-Verbindung, und anschliessender Umsetzung dieser Gemischten-Anydrid-Verbindung mit einem Alkylester des L-Phenylalanins oder jedem anderen Aminosäure-Ester unter Bildung des gewünschten Dipeptids und insbesondere der Methylester-Süssstoff-Verbindung hergestellt werden.

Nach der Erfindung wird zunächst das Dialkali-Salz der L-Asparaginsäure gebildet, und zwar üblicherweise durch Reaktion eines Alkali-Hydroxids, wie zum Beispiel ein Alkalimetall-Hydroxid von Kalium, Natrium oder Lithium, in einer alkoholischen Lösung, wie Methanol, Äthanol oder ein anderes niedriges Alkanol, mit L-Asparagin-Säure. Insbesondere bevorzugt wird die Reaktion der L-Asparagin-Säure in einer Methanol-Kaliumhydroxid-Lösung zur Bildung des Dikaliumsalzes.

Nach der Bildung des Dialkali-Salzes der Asparaginsäure wird dessen Amino-Gruppe geschützt durch Reaktion eines der Wasserstoffatome der Amino-Gruppe, üblicherweise in situ, mit einer geeigneten organischen Verbindung. Für die Reaktion mit der Amino-Gruppe und den Schutz derselben steht eine Vielzahl organischer Verbindungen zur Verfügung. Die eingesetzte Amino-Schutz-Verbindung sollte, üblicherweise in situ, mit der Amino-Gruppe reagiert werden, um die Amino-Gruppe vor einer weiteren Reaktion während der folgenden Umsetzung mit dem Halogen-Ester zur Bildung des Gemischten-Anhydrids, zu schützen.

Die Amino-Schutzgruppe sollte im Anschluss an die Kondensationsstufe durch Senkung des pH-Wertes auf einen Säure-pH-Wert oder mittels Hydrierung entfernt werden, damit die freie Amino-Gruppe und die freie Carboxyl-Gruppe der gewünschten Asparaginsäure-Verbindung rückgebildet werden. So kann beispielsweise die Amino-Gruppe durch Reaktion mit einem Carbobenzoxyhalogenid, wie ein Carbobenzoxychlorid gemäss der verwandten Patentanmeldung geschützt werden. Jedoch verlangt die Verwendung dieser Verbindung eine gesonderte Hydrierungsstufe, wie bei dem verwandten Verfahren. Eine bevorzugte Verbindung zum Schutz der Amino-Gruppe besteht in einer Alkyl-Acetoacetat-Verbindung, wie zum Beispiel Methyl-, Äthyl- und Propylacetoacetat.

Das aminogruppengeschützte Dialkali-Aspartat wird mit einem Halogenester in einer Menge reagiert, welche ausreicht, um hierdurch eines der Alkalimetallatome unter Bildung des Monoalkali-Salzes einer Gemischten-Anhydrid-Verbindung zu entfernen. In der Gemischten-Anhydrid-Verbindung ist somit sowohl die Beta-Carboxyl-Gruppe als Alkali-Salz als auch die Amino-Gruppe geschützt. Üblicherweise besteht der verwen-

dete Halogenester aus einem $C_1$- bis $C_6$-Alkylhalogenester, wobei das Halogen Chlor oder Brom sein kann und sich der Halogenester von einer $C_1$-bis $C_4$-Säure ableitet. Die bevorzugte Verbindung ist ein Alkylchlorameisensäureester, wie Methyl-, Äthyl-, Propyl-, Chlorameisensäureester.

Die Gemischte-Anhydrid-Verbindung wird sodann in einer Kondensationsreaktion unter Alkalibedingungen umgesetzt; zum Beispiel bei etwa pH 8–9 und bei niedriger Temperatur von beispielsweise unter −20°C, und zwar mit einem Alkylester des L-Phenylalanins, und zwar insbesondere dem Methylester. Der pH-Wert der Reaktionsmischung wird anschliessend auf einen pH-Wert von 2,0 oder darunter – üblicherweise 1,5 und weniger – reduziert, damit die Amino- und die Carboxylgruppe wieder freigesetzt werden. Dadurch kommt es zur Bildung eines Alpha-Beta-Gemisches des gewünschten Alkyl-Esters des L-Aspartyl-L-Phenylalanins. Die Methode ermöglicht die direkte Herstellung des Alpha-Beta-Gemisches der Dipeptid-Verbindung in guter Ausbeute, ohne störende Nebenprodukte, sowie zu einem niedrigen Kostenpreis, da preiswerte Ausgangsmaterialien verwendet werden können.

Somit bietet die Erfindung die Möglichkeit der direkten Herstellung des gewünschten Alpha-Dipeptid-Esters in guter Ausbeute durch Reaktion des Dialkali-Salzes der Asparaginsäure mit geschützter Amino-Gruppe, in situ, mit einem Halogenester zum Alkali-Salz eines Gemischten-Anhydrids, sowie durch Reaktion der Gemischten-Anhydrid-Verbindung mit dem L-Phenylalanin-Methylester und nachfolgender Freisetzung der geschützten Gruppen durch Reduzieren des pH-Wertes. Dieses Verfahren liefert gleichzeitig sowohl Alpha- als auch Beta-Dipeptid-Ester. Das gewünschte Alpha-Isomere wird vorzugsweise in einer grösseren Menge gebildet, und zwar in Abhängigkeit von den angewandten Reaktionsbedingungen. Üblicherweise umfassen solche Reaktionsbedingungen die Durchführung der Kondensationsreaktion bei Temperaturen von −20°C bis −60°C; zum Beispiel −30°C bis −40°C, und einem pH-Wert von 8 bis 9, üblicherweise 8,5 bis 8,9, mit anschliessender Rückbildung der freien Amino- und Carboxylgruppen durch Senkung des pH-Wertes. Das erhaltene Reaktionsgemisch besteht nahezu gänzlich aus Alpha- und Beta-Dipeptid, wobei das Alpha-Isomere in grösserer Menge vorliegt. Der unerwünschte Beta-Dipeptid-Ester kann mühelos von dem Alpha-Isomeren anhand der bekannten Methoden getrennt werden. Das Verfahren erfordert nicht viele Reaktionsstufen und keine kostspieligen Reagenzien und kann ohne weiteres für die industrielle Anwendung eingesetzt werden.

Die Prozessbedingungen einer jeden Verfahrensstufe können unterschiedlich sein. Bei der Bildung des Alkali-Salzes der Asparaginsäure sollte jedoch eine ausreichende stöchiometrische Menge an Alkali-Hydroxid für die Herstellung des Dialkali-Aspartats vorhanden sein, sowie ausreichende Mengen der Amino-Schutz-Verbindung angewandt werden, um einen Schutz der Amino-Gruppe der Dialkali-Aspartat-Verbindung zu gewährleisten. Danach sollte eine ausreichende stöchiometrische Menge des Halogenesters eingesetzt werden zur Bildung des Monoalkali-Salzes der Gemischten-Anhydrid-Verbindung. Im Anschluss daran erfolgt die Kondensationsreaktion unter Verwendung einer stöchiometrischen Konzentration des L-Phenylalaninesters unter Alkali-Bedingungen bei niedrigen Temperaturen. Zur Bildung des gewünschten Dipeptids wird dann der pH-Wert des Reaktionsgemisches gesenkt, um die Amino-Gruppe und die Carboxyl-Gruppe wieder freizusetzen.

Die Erfindung ist nachstehend in Form eines allgemeinen Schemas dargestellt:

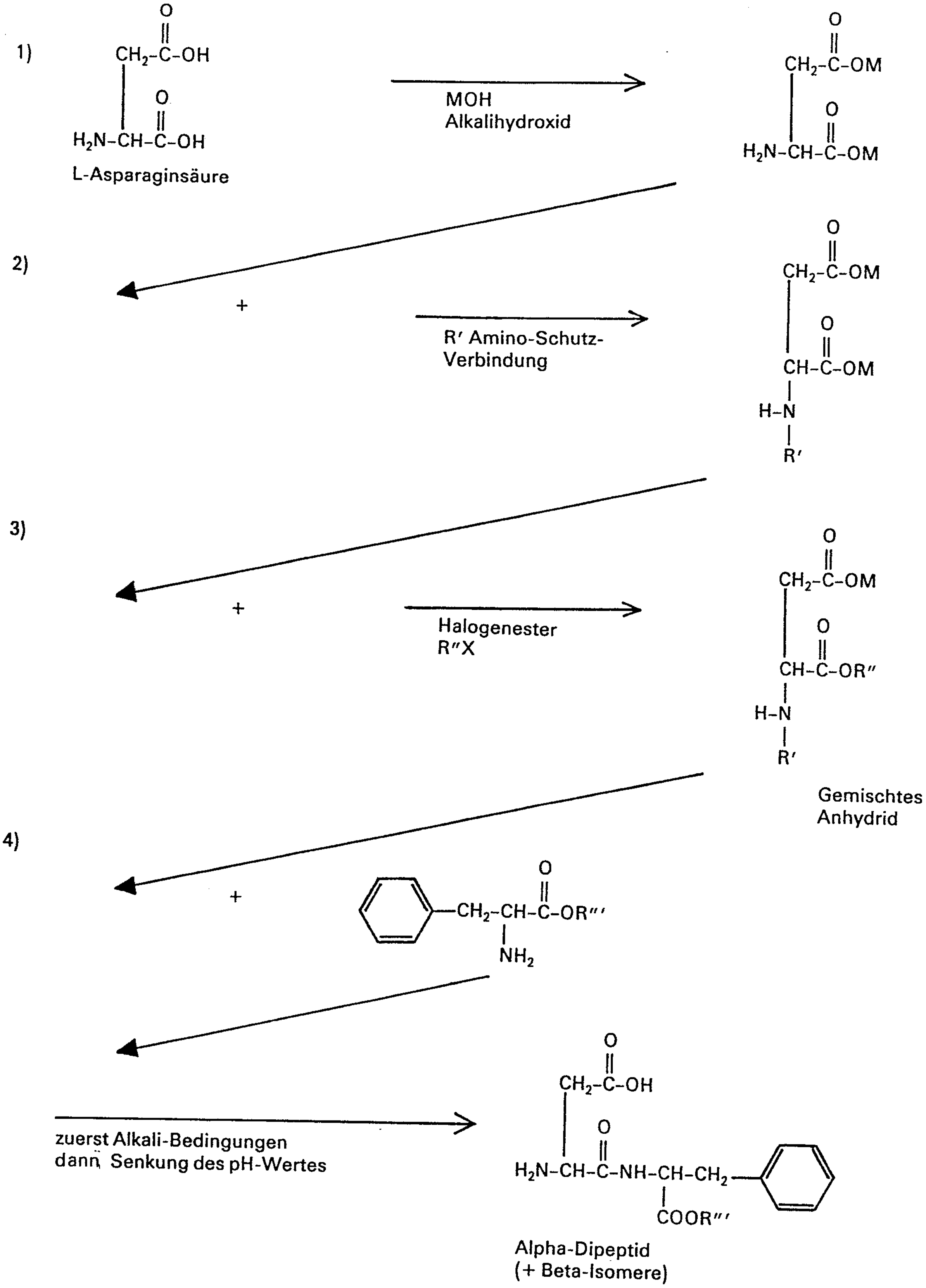


M ist hierbei ein Alkalimetall; R′ eine Amino-Schutz-Verbindung; R″ eine Ester-Gruppe des Halogenesters; X ein Halogen und R‴ eine Alkyl-Gruppe.

Eine bevorzugte, spezifische Reaktionsfolge, unter Verwendung einer spezifischen Verbindung, ist wie folgt dargestellt:

1)

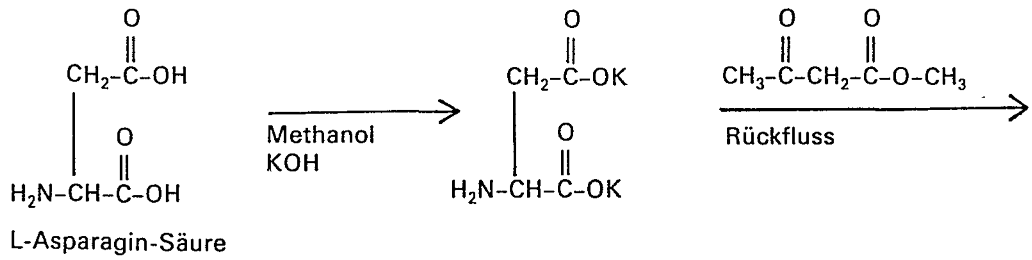
Methanol
KOH
Rückfluss
L-Asparagin-Säure

2)

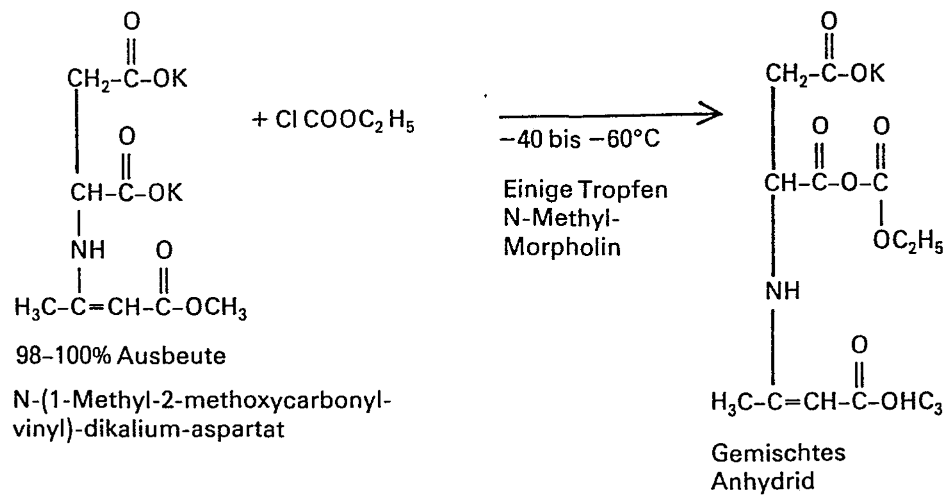
−40 bis −60°C
Einige Tropfen
N-Methyl-
Morpholin
98–100% Ausbeute
N-(1-Methyl-2-methoxycarbonyl-
vinyl)-dikalium-aspartat
Gemischtes
Anhydrid

3)

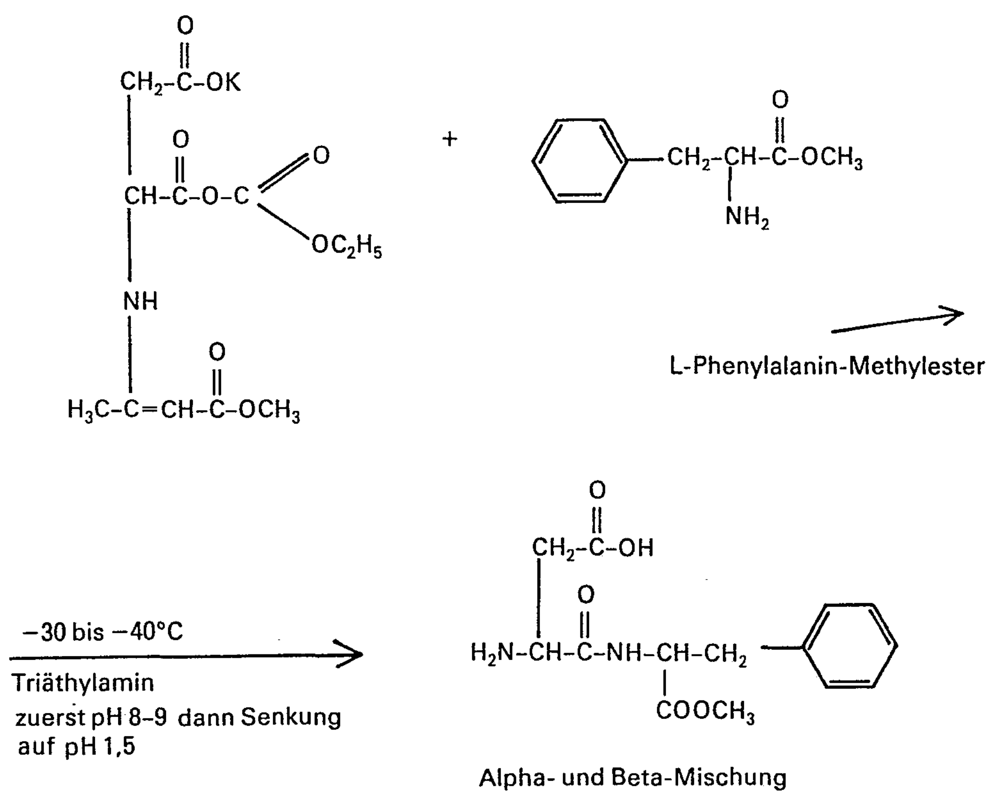
L-Phenylalanin-Methylester
−30 bis −40°C
Triäthylamin
zuerst pH 8–9 dann Senkung
auf pH 1,5
Alpha- und Beta-Mischung

Die Methode gemäss der Erfindung wird nachstehend anhand von bevorzugten Versuchsbeispielen beschrieben. Fachleute können indessen verschiedene Änderungen und Umstellungen der gezeigten Arbeitsweise im Sinne und entsprechend dem Zweck der Erfindung vornehmen.

Beispiel 1
Herstellung des Dikalium-L-Aspartyl N-3-Ketobutenoat
In einem Dreihalskolben wurden 360 ml Methanol (über Molekularsieb destilliert) sowie 44,8 g Kalium-Hydroxid gegeben. Der Kolben wurde mit Rückflusskühler und Rührwerk versehen. Der Inhalt wurde unter Rühren auf 45°C erhitzt, bis eine klare Lösung vorlag. Nach Abkühlen auf Raumtemperatur wurden langsam und unter ständigem Rühren 45,2 g L-Asparaginsäure hinzugegeben und der Inhalt wurde dann 15 Minuten lang am Rückfluss gekocht. Nach Abkühlung auf Raumtemperatur wurden langsam 40,1 ml Methylacetoacetat in 197 ml wasserfreiem Methanol hinzugefügt und 30 Minuten am Rückfluss erhitzt.

Das Methanol wurde vollständig unter Vakuum abdestilliert und der Rückstand zu 500 ml 2-Propanol hinzugegeben. Der gebildete weisse Niederschlag wurde abgefiltert und über Kalium-Hydroxid in einem Vakuumexsiccator getrocknet. Die Gesamtausbeute betrug 97 bis 98%.

Beispiel 2
Kondensation des Dikaliumsalzes des L-Aspartyl N-3-Ketobutenoat mit dem Phenylalanin-Ester
4,6 g Dikalium-L-Aspartyl N-3-Ketobutenoat wurden in einen 500 ml Dreihalskolben, ausgerüstet mit Rührwerk, Thermometer und einem Kalziumchlorid-Rohr, gegeben, Azeton (40 ml) hinzugefügt und bei −55°C 10 Minuten lang gerührt.

Unter weiterem Rühren wurde Äthyl-Chlorameisensäureester (2,0 ml) zugegeben und anschliessend 5 Tropfen N-Methyl-Morpholin. Die Temperatur wurde auf −30°C erhöht und die Mischung bei −30°C bis −40°C 30 bis 60 Minuten lang, vorzugsweise 45 Minuten, gerührt. Danach wurde sie auf −70°C abgekühlt und durch ein Celite-Filter filtriert. Das Filtrat wurde bei −55°C aufbewahrt.

Zu der auf −55°C temperierten Gemischten-Anhydrid-Lösung wurde Phenylalanin-Methylester-Hydrochlorid (4,2 g) in 10 ml Wasser und mit Triäthylamin neutralisiert hinzugefügt und der pH-Wert auf 8,5 bis 8,9 eingestellt. Der Inhalt wurde bei −40°C bis −30°C 1½ Stunden lang gerührt. Danach wurden zunächst 25 ml Wasser und anschliessend konzentrierte Salzsäure hinzugegeben und hierdurch der pH-Wert auf 1,5 gebracht. Die Mischung wurde erneut bei 0°C 10 Minuten lang gerührt, anschliessend mit Dichlormethan extrahiert (2 × 70 ml) und die wasserhaltige Schicht auf pH 4,1 eingestellt.

Die erhaltene wässrige Lösung wurde auf einer Silikagel-Säule chromatographiert, um reinen Alpha-L-Aspartyl-L-Phenylalanin-Methylester herzustellen. Aus der wässrigen Lösung kristallisierte bei 0°C Alpha-L-Aspartyl-L-Phenylalanin-Methylester.

Das Produkt wurde durch Schmelzpunkt (235°C bis 236°C) Mischschmelzpunkt und Dünnschichtchromatographie-Vergleich mit einer authentischen, handelsüblichen Probe identifiziert.

Die Ausbeuten an Alpha- beziehungsweise Beta-Isomeren betrugen 60 beziehungsweise 35%.

**Patentansprüche**

1. Verfahren zur Herstellung eines Alpha-L-Aspartyl-L-Phenylalanin-Alkyl-Esters, gekennzeichnet durch:

a) Reaktion eines Dialkali-Salzes der L-Asparaginsäure mit geschützter Aminogruppe mit einer organischen Halogen-Ester-Verbindung zur Bildung des Monoalkali-Salzes einer Gemischten-Anhydrid-Verbindung;

b) Kondensation der Gemischten-Anhydrid-Verbindung mit einem Alkylester des L-Phenylalanins unter Alkalibedingungen und Senkung des pH-Wertes nach der Kondensation auf Säurebedingungen, um die Amino- und Carboxylgruppen freizusetzen, zur Bildung eines Gemisches, im wesentlichen bestehend aus dem Alpha- und Beta-Alkylester des L-Aspartyl-L-Phenylalanins; und

c) Abtrennung des Alpha-Isomeren aus dem Alpha- und Beta-Gemisch.

2. Verfahren nach Anspruch 1, dadurch gekennzeichnet, dass das eingesetzte Alkali-Salz ein Kaliumsalz ist.

3. Verfahren nach Anspruch 1, gekennzeichnet durch die Reaktion eines Alkyl-Acetoacetats mit der Amino-Gruppe, in situ, zum Schutz der Amino-Gruppe, bevor die Reaktion mit der Halogen-Ester-Verbindung erfolgt.

4. Verfahren nach Anspruch 1, dadurch gekennzeichnet, dass die Halogen-Ester-Verbindung ein Alkyl-Halogenformiat ist.

5. Verfahren nach Anspruch 4, dadurch gekennzeichnet, dass der Halogen-Ester ein Alkyl-Chlorameisensäureester ist.

6. Verfahren nach Anspruch 1, dadurch gekennzeichnet, dass der Alkyl-Ester des L-Phenylalanins der Methylester ist.

7. Verfahren nach Anspruch 1, dadurch gekennzeichnet, dass die Kondensation bei einem pH-Wert von 8 bis 9 unter einer Temperatur von −20°C bis −60°C erfolgt.

8. Verfahren nach Anspruch 1, dadurch gekennzeichnet, dass der pH-Wert im Anschluss an die Kondensation auf 1,5 oder weniger gesenkt wird.

9. Verfahren zur Herstellung eines Alpha-L-Aspartyl-L-Phenylalanin-Methylesters, gekennzeichnet durch:

a) Reaktion von L-Asparagin-Säure mit einem alkoholischen Alkalimetall-Hydroxid zur Bildung des Dialkali-Salzes der Asparagin-Säure;

b) Reaktion der freien Amino-Gruppe des Alkali-Salzes, in situ, mit einem Alkyl-Acetoacetat zum Schutz der Amino-Gruppe;

c) Reaktion des Dialkali-Salzes der Asparaginsäure mit geschützter Aminogruppe mit einem Alkyl-Halogenformiat zur Bildung eines Monoalkali-Salzes einer Gemischten-Anhydrid-Verbindung;

d) Kondensation der Gemischten-Anhydrid-Verbindung mit einem Methylester des L-Phenylalanins bei einem pH-Wert von etwa 8 bis 9 und einer Temperatur von unter −20°C;

e) Senkung des pH-Wertes der Reaktionsmischung von Schritt d) auf Säurebedingungen zur Freisetzung der Amino- und Carboxylgruppen und Bildung eines Gemisches von Alpha- und Beta-Methylester des L-Aspartyl-L-Phenylalanins; und

f) Trennung des Alpha- und Beta-Methylesters, um den Alpha-L-Aspartyl-L-Phenylalanin-Methylester zu erhalten.

## Revendications

1. Procédé de fabrication d'un alpha-L-aspartyl-L-phénylalanine-alkyl-ester, caractérisé:

a) par la réaction d'un sel double alcalin de l'acide L-aspartique avec un groupe aminé protégé, avec une combinaison organique halogène-ester pour former le sel monoalcalin d'une combinaison d'anhydride mixte;

b) par la condensation de la combinaison d'anhydride mixte avec un alkyl-ester de la L-phénylalanine en milieu alcalin et abaissement de la valeur du pH après condensation en milieu acide, cela pour libérer les groupes aminés et les groupes carboxyles en vue de former un mélange se composant essentiellement d'alpha- et de bêta-alkyl-ester de L-aspartyl-L-phénylalanine et

c) par la séparation des isomères alpha du mélange alpha et bêta.

2. Procédé, conforme à la revendication 1, caractérisé par le fait que le sel alcalin ajouté est un sel de potassium.

3. Procédé, conforme à la revendication 1, caractérisé par la réaction d'un alkyl-acétoacétate avec le groupe amine «in situ», cela pour protéger le groupe aminé avant que ne se produise la réaction avec la combinaison halogène-ester.

4. Procédé, conforme à la revendication 1, caractérisé par le fait que la combinaison halogène-ester est un alkylformiate d'halogène.

5. Procédé, conforme à la revendication 4, caractérisé par le fait que le halogène-ester est un alkylester de l'acide chloroformique.

6. Procédé, conforme à la revendication 1, caractérisé par le fait que l'alkylester de la L-phénylalanine est le méthylester.

7. Procédé, conforme à la revendication 1, caractérisé par le fait que, pour une valeur du pH, comprise entre 8 et 9, la condensation se produit au-dessous d'une température, comprise entre −20° et −60°C.

8. Procédé, conforme à la revendication 1, caractérisé par le fait qu'à la suite de la condensation, le pH est abaissé à une valeur égale ou inférieure à 1,5.

9. Procédé de fabrication d'un alpha-L-aspartyl-L-phénylalanine-méthylester, caractérisé:

a) par la réaction de l'acide L-aspartique avec un hydroxyde alcoolique d'un métal alcalin pour former le sel double alcalin de l'acide aspartique;

b) par la réaction du groupe libre aminé du sel alcalin «in situ» avec un alkyl-acétoacétate pour protéger le groupe aminé.

c) par la réaction du sel double alcalin de l'acide aspartique avec le groupe aminé protégé, avec un alkyl-formiate d'halogène pour former un sel monoalcalin d'une combinaison d'anhydride mixte;

d) par la condensation de la combinaison d'anhydride mixte avec un méthylester de la L-phénylalanine pour une valeur du pH comprise entre 8 et 9 ainsi que pour une température inférieure à −20°C.

e) par l'abaissement de la valeur du pH du mélange en réaction en milieu alcalin pour libérer les groupes aminés et les groupes carboxyles et former un mélange d'alpha et de bêta-méthylester de L-aspartyl-L-phénylalanine et

f) par la séparation de l'alpha et du bêta-méthylester pour obtenir l'alpha-L-aspartyl-phénylalanine-méthylester.

## Claims

1. A method for preparing an Alpha-L-aspartyl-L-phenylalanine alkyl ester, characterised in

a) reacting a divalent alkali salt of L-aspartic acid, having an amino-protective group, with an organic halo ester compound, to form a monovalent, alkali-salt, mixed, anhydride aspartate compound; and

b) condensing the mixed, anhydride, aspartate compound with an alkyl ester of L-phenylalanine under alkaline conditions and reducing the pH to an acidic condition after condensation to free the amino and carboxyl groups, to form a mixture consisting essentially of the alpha and beta alkyl ester of L-aspartyl-L-phenylalanine; and

c) separating the alpha isomer from the alpha and beta mixture.

2. The method of claim 1, characterised in that the alkali salt is a potassium salt.

3. The method of claim 1, characterised in reacting an alkyl acetoacetate with the amino group, in situ, to protect the amino group, prior to reacting with the halo ester compound.

4. The method of claim 1, characterised in that the halo ester compound comprises an alkyl haloformate.

5. The method of claim 4, characterised in that the halo ester is an alkyl chloroformate.

6. The method of claim 1, characterised in that the alkyl ester of L-phenylalanine is the methylester.

7. The method of claim 1, characterised in that the condensation is carried out at a pH of about 8 to 9 and at a temperature of −20°C to −60°C.

8. The method of claim 1, characterised in that the pH after condensation is reduced to 1.5 or less.

9. A method for preparing an Alpha-L-aspartyl-L-phenylalanine methylester, characterised in

a) reacting L-aspartic acid with an alcoholic metal alkali hydroxide, to form the divalent alkali salt of aspartic acid;

b) reacting the free-amino group of the alkali salt, in situ, with an alkyl acetoacetate, to protect the amino group;

c) reacting the amino-protective, divalent alkali salt of aspartic acid with an alkyl haloformate, to form a monovalent, mixed, anhydride aspartate compound;

d) condensing the mixed, anhydride, aspartate compound with a methylester of L-phenylalanine at a pH of about 8 to 9 and at a temperature of less than about −20°C;

e) reducing the pH of the reaction mixture of step d) to an acidic condition to free the amino and carboxyl groups, to form a mixture of the alpha and beta methylester and

f) separating the alpha and beta methylester, to recover the Alpha-L-aspartyl-L-phenylalanine methylester.